# EUROPÄISCHE PATENTANMELDUNG

(11) **EP 1 055 408 A1**
(43) Veröffentlichungstag der Anmeldung: **29.11.2000**
(21) Anmeldenummer: 00110882.8
(22) Anmeldetag: 23.05.2000
(51) Int. Cl.: A61K 7/06, A61K 7/48, A61K 31/35

(54) **Sorbatkonservierte Mittel, Verfahren zu deren Herstellung und ihre Verwendung**

(30) Priorität: 26.05.1999 DE 19923838; 22.06.1999 DE 19928495
(71) Anmelder: Nutrinova Nutrition Specialties & Food Ingredients GmbH, 65929 Frankfurt am Main (DE)
(72) Erfinder: Schmidt, Ralf, 90478 Nürnberg (DE)
(74) Vertreter: Schweitzer, Klaus, Dr.

(57) **Zusammenfassung**

Die Erfindung betrifft ein Mittel, das Sorbinsäure und/oder ein oder mehrere Sorbate sowie ein Flovonoid und/oder Flavonoidderivat enthält, ein Verfahren zu deren Herstellung und ihre Verwendung.

## Beschreibung

Die Erfindung betrifft sorbatkonservierte Mittel, Verfahren zu ihrer Herstellung sowie ihre Verwendung, insbesondere als farbstabilisierte kosmetische und pharmazeutische Mittel.

Kosmetische Mittel sind Stoffe oder Zubereitungen aus Stoffen, die dazu bestimmt sind, äußerlich am Menschen oder in Körperhöhlen (z.B. in der Mundhöhle) zur Reinigung, Pflege oder zur Beeinflussung des Aussehens oder des Körpereigengeruchs oder zur Vermittlung von Geruchseindrücken angewendet zu werden. Pharmazeutische Mittel sind Stoffe oder Zubereitungen aus Stoffen, die vornehmlich dazu bestimmt sind, Krankheiten, Leiden, Körperschäden oder krankhafte Beschwerden zu lindern oder zu beseitigen.

Kosmetische Mittel sind beispielsweise Haar- und Körperreinigungsprodukte wie Shampoos, Duschgele oder Waschlotionen, Cremes, Lotionen und Gele zur Hautpflege und zum Schutz gegen Sonnenbestrahlung, Mund- und Zahnpflegeprodukte, dekorative Kosmetik, Mittel zur Selbstbräunung, wasser- alkohol- oder tensidhaltige Tücher zur Reinigung oder Erfrischung und weitere Produkte im Sinne der oben genannten Definition.

Pharmazeutische Mittel im Sinne dieser Erfindung sind wässrige oder alkoholhaltige Lösungen, Gele oder Emulsionen aus einer Wasser- und einer Lipidphase, die pharmazeutisch wirksame Stoffe enthalten. Beispiele sind oral applizierbare Arzneimittel, Salben, Cremes, Augen- oder Nasentropfen, Sprays, Tinkturen, Injektionslösungen und weitere mehr.

Typische Inhaltsstoffe kosmetischer und pharmazeutischer Mittel sind Wasser, Tenside und Co-Tenside, Öle, Fette, Wachse, Emulgatoren, Solubilisatoren, Filmbilder, Polymere, Konditioniermittel, Konsistenzgeber, Verdicker, Gelbildner, Farbstoffe, Pigmente, Perlglanzmittel, Duftstoffe, Lichtschutzfilter, Deowirkstoffe, Feuchthaltemittel, Naturstoffextrakte wie Kräuter, Pflanzenextrakte oder ätherische Öle, Lösungsmittel, Abrasiva und weitere mehr. Des weiteren finden verschiedenste Wirkstoffe in solchen Produkten Verwendung, beispielsweise hautglättende, entzündungshemmende, schmerzstillende, antibakterielle, antifungale und antivirale Stoffe.

Kosmetische und pharmazeutische Mittel, insbesondere wasserhaltige, oder solche die durch den Zutritt von Wasser oder Luftfeuchtigkeit Wasser aufnehmen können, enthalten in der Regel einen oder mehrere Konservierungsstoffe, die das Mittel während der Lagerung und während des Gebrauchs gegen Befall mit verderbniserregenden oder krankheitserregenden Mikroorganismen schützen.

Sorbinsäure (2,4-Hexadiensäure) und ihre Salze, im besonderen das gut wasserlösliche Kaliumsalz, werden seit vielen Jahren zur Konservierung von Lebensmitteln, sowie kosmetischen und pharmazeutischen Mitteln weltweit verwendet. Bei Sorbinsäure handelt es sich um eine ungesättigte Fettsäure, die sich durch besondere physiologische Verträglichkeit auszeichnet. Sorbinsäure wird im menschlichen Körper analog zu seiner Fettsäure verstoffwechselt, akkumuliert nicht und wird von den wissenschaftlichen Beratungsgremien der Weltgesundheitsorganisation und der Europäischen Union als sicher eingestuft. Der von beiden Gremien für Sorbinsäure festgelegte ADI-Wert, der als Maß für die physiologische Unbedenklichkeit von Lebensmittelzusatzstoffen gewertet werden kann, beträgt 0 bis 25 mg/kg Körpergewicht und Tag und ist damit der mit Abstand höchste ADI-Wert aller Konservierungsstoffe. Sorbinsäure und Sorbate gelten als nicht allergen und werden daher auch in keiner der bekannten Allergiedatenbanken (z.B. Leatherhead Food Tolerance Databanks Project) erwähnt. Bezüglich der Verwendung in kosmetischen Mitteln stuft das CIR-Expert Panel der CTFA basierend auf den zu Sorbinsäure und Sorbaten bekannten toxikologischen und allergologischen Daten diese Konservierungsstoffe als "sicher" ein. Sorbinsäure und Sorbate sind weltweit in der überwiegenden Zahl der Länder zur Konservierung von kosmetischen Mitteln zugelassen.

Die Wirksamkeit der Sorbinsäure richtet sich vor allem gegen Hefen und Schimmelpilze, sowie gegen zahlreiche Bakterien. Die Wirksamkeit der Sorbinsäure hängt vom undisoziierten Anteil und damit vom pH-Wert des zu konservierenden Gutes ab. Aufgrund des hohen pK-Wertes von 4,76 ist Sorbinsäure im Gegensatz zu anderen Konservierungsstoffen auf Basis organischer Säuren auch zur Konservierung von schwach sauren Gütern (bis pH 6,5) geeignet.

In fester Form sind Sorbinsäure und die meisten Sorbate, insbesondere Kaliumsorbat und Calciumsorbat, stabil. In wässriger Lösung, in Lebensmitteln und in kosmetischen Mitteln unterliegt Sorbinsäure jedoch oxidativen Einflüssen. Besonders durch oxidative Spaltung der Doppelbindungen können Aldehyde und Ketone entstehen, die die Ursache für Off-flavours sein können. Polymerisationsprodukte dieser Aldehyde können ebenso für farbliche Veränderungen, im besonderen Bräunungsreaktionen, verantwortlich sein, wie die Reaktionsprodukte dieser Aldehyde mit Aminosäuren bzw. anderen primären oder sekundären Aminogruppen. Derartige Produkte werden als Maillard-Produkte bezeichnet und sind vielfach für Farbveränderungen in kosmetischen Mitteln und Lebensmitteln verantwortlich.

Gerade in kosmetischen Mitteln, für die deutlich längere Lagerzeiten angenommen werden müssen als für die meisten Lebensmittel und die oxidativen Einflüssen ausgesetzt sind, ist eine sorbatinduzierte Braunverfärbung beschrieben (Domsch, A. (1994): Die kosmetischen Präparate, Band 2, wässrige und tensidhaltige Formulierungen, 4. Auflage, S. 329, Augsburg, Verlag für chemische Industrie).

Der Mechanismus der Oxidation von Sorbinsäure und entsprechende Stabilisierungsmaßnahmen sind vielfach Gegenstand wissenschaftlicher Untersuchungen gewesen (Arya, S. (1980): Stability of sorbic acid in aqueous solutions, Journal Agric. Food Chem. 28, 1246-1249; Arya, S., Thakur, B. (1988): Degradation products of sorbic acid in aqueous solutions, Food Chem. 29, 41-49; Ledward, D. (1990): Stability of sorbic acid in intermediate moisture systems, Food Add. Contam. 7, 677 - 683; Merciades, M., Mohammed, K., Maniere, F. (1992): Stabilized sorbic acid or salt thereof, EP-A 0 595 576; Thakur, B., Singh, R., Arya, S. (1994): Chemistry of sorbates - a basic perspective. Food Rev. Intern. 10, 71 - 91).

Da zumindest der erste Schrill der Reaktionskette, die im weiteren Verlauf zur Bildung gefärbter Verbindungen führt, wahrscheinlich einen oxidativen Angriff auf eine der im Sorbinsäuremolekül enthaltenen Doppelbindungen darstellt, erscheint die Mitverwendung eines antioxidativ wirkenden Bestandteils nötig. Es ist allerdings bekannt, daß gängige Antioxidantien wie z.B. Propylgallat oder BHA (t-Buthylmethoxyphenol), die aufgrund ihrer hohen antioxidativen Kapazität in zahlreichen kosmetischen Produkten und auch in manchen Lebensmitteln gebräuchlich sind, auf sorbathaltige kosmetische oder pharmazeutische Formulierungen nicht farbstabilisierend wirken.

Im Rahmen der o.g. Untersuchungen wurde teilweise versucht, die beschriebenen sorbatinduzierten Verfärbungen und geruchlichen Veränderungen von Lebensmitteln durch den Zusatz von Metallionen (insbesondere Mangan) im Konzentrationsbereich von 0,1 bis 5 ppm zu vermindern. Metallionen sind jedoch nur in einem äußerst engen Dosierungsbereich antioxidativ wirksam. US-A 5,354,902 beschreibt die Farbstabilisierung sorbathaltiger wässriger Systeme unter Verwendung von Mangan-Ionen. Insgesamt scheint der Zusatz von Schwermetall-lonen zur Lösung des Verfärbungsproblems aber wenig geeignet, da von den Schwermetallen sowohl toxische, wie auch ökologische ungünstige Wirkungen ausgehen. Hinzu kommt, daß die genannten Schwermetalle je nach gewählter Konzentration auch prooxidativ wirken können.

US-A 2,866,817 beschreibt die Stabilisierung wässriger Lösungen von Sorbinsäure und ihren Salzen, insbesondere Na-Sorbat, mit 0,0005 bis 0,5 % Glucono-delta-lacton. In Kombination mit typischen Bestandteilen kosmetischer Mittel wie z.B. Proteinhydrolysaten, Ethanolamiden oder Amidopropylbetainen, die eine Verfärbung zusätzlich begünstigen, führt diese Methode jedoch nicht zum Erfolg. Na-Sorbat hat zudem aufgrund seiner begrenzten Stabilität bereits in fester Form als Konservierungsstoff für Lebensmittel, sowie kosmetische und pharmazeutische Mittel keine Bedeutung.

Die bisher am häufigsten durchgeführte protektive Maßnahme zur Verminderung sorbatinduzierter Verfärbungen ist die Milverwendung komplexierender Agenzien (z.B. EDTA (Ethylendiamintetraacetat) und Citronensäure oder deren Salze), die durch Komplexierung prooxidativ wirkender Metallionen sorbatinduzierte Verfärbungen verlangsamen.

Diese Maßnahme ist besonders dann von Vorteil, wenn eine Verunreinigung durch Spuren prooxidativ wirksamer Metallionen im kosmetischen Produkt gegeben ist. Unter ungünstigen Bedingungen (z.B. erhöhte Temperaturen oder intensive Tageslichtbestrahlung des sorbathaltigen Produktes) treten Verfärbungen jedoch auch in Abwesenheit von solchen prooxidativen Faktoren ein, so daß Komplexbildner allein keinen ausreichenden Schutz bieten können.

DE-A 195 04 999 beschreibt die Verwendung von Allantoin mit oder ohne Citronensäure oder eines deren Salze zur Verminderung sorbatinduzierter Verfärbungen in Kosmetika und Lebensmitteln. Diese Lösung besitzt jedoch den Nachteil, daß die farbstabilisierende Wirkung in Verbindung mit einigen häufig verwendeten Inhaltsstoffen kosmetischer Produkte nicht ausreichend ist. Hier sind insbesondere solche Inhaltsstoffe von Bedeutung, die aufgrund ihrer Herstellung Restgehalte an freien Aminen enthalten oder selbst Moleküle mit primären oder sekundären Amimogruppen darstellen. Beispiele für in dieser Weise nachteilig wirkende Inhaltsstoffe sind Amidopropylbetaine, Proteinhydrolysate oder Ethanolamide, sowie deren Fettsäure-Ester.

Insbesondere unter intensiver Bestrahlung durch Tageslicht werden an das farbstabilisierende Prinzip einer sorbathaltigen kosmetischen oder pharmazeutischen Formulierung besondere Anforderungen gestellt, die alleine durch den Einsatz von Allantoin in Verbindung mit Citronensäure und Citraten nur teilweise erfüllt werden können. Die Verfärbung entsprechender Produkte wird durch den Zusatz von Allantoin in Verbindung mit Citronensäure und Citraten zwar vermindert, bleibt jedoch optisch wahrnehmbar.

Daher besteht auch weiterhin ein Bedarf für farbstabilisierende Additive, die in der Lage sind, sorbatinduzierte Verfärbungen in kosmetischen oder pharmazeutischen Mitteln zu unterbinden oder diese selbst unter ungünstigen Bedingungen wie erhöhter Licht- und Wärmebelastung merklich zu verringern.

Eine effektive Farbstabilisierung gelingt überraschenderweise dadurch, daß man den sorbathaltigen Mitteln ein Flavonoid zusetzt, insbesondere ein Flavon oder Flavonol, bevorzugt ein wasserlösliches 3- oder 7-Glycosid eines Flavonols, besonder bevorzugt Rutin, das Rutinosid des Quercetin. Die erfindungsgemäßen Flavonoide sind in der Regel pflanzlicher Herkunft.

Flavonoide sind selbst schwach gelbliche Produkte, die z.B. in der Naturfärberei Verwendung finden. Auf dem pharmazeutischen Feld wird einigen Flavonoiden bzw. ihren Glycosiden protektive Wirkung gegen Erkrankungen der Venen (Biflavonoide wie Hesperidin und Rutin) und des Leberparenchyms (Silybin) zugeschrieben, außerdem eine Verbesserung der coronaren Durchblutung (Weißdorn-Extrakt) und diuretische (Birkenblätter-) oder spasmolytische (Kamillen-Drogen) Wirkungen. (Römpp Lexikon Chemie, 10. Auflage, Thieme Verlag, Stuttgart, New York).

Glycoside im Sinne dieser Erfindung sind Verbindungen der Flavonoide mit Mono- oder Oligosacchariden, bestehend aus den monomeren Bausteinen Glucose, Rhamnase, Arabinose, Galactose, Xylose oder anderen Monosacchariden, am C-Atom 3 oder 7 des Flavonoids, wie sie in heimischen Obstarten und Südfrüchten häufig vorkommen:

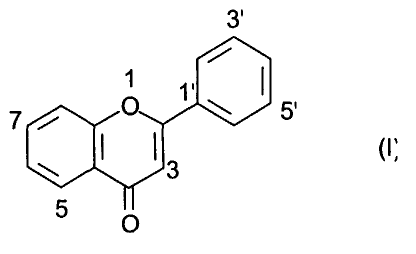

Formel (I) zeigt den Grundkörper eines Flavons (3=H) bzw. Flavonols (3=OH, 4'=OH).
Beispiele für erfindungsgemäß wirksame Flavonoide sind die Flavone
Apigenin (5=OH, 7=OH)
Luteolin (5=OH, 7=OH, 3'=OH)
Diosmetin (4'=OCH₃, 3'=OH)
Chrysoeriol (3'=OCH₃, 4'=OH)
   sowie die Flavonole
Kämpferol (5=OH, 7=OH)
Quercetin (5=OH, 7=OH, 3'=OH)
Morin (5=OH, 7=OH, 2'=OH)
Robinetin (7=OH, 3'=OH, 5'=OH)
Gossypetin (5=OH, 7=OH, 3'=OH, 8=OH)
Myricetin (5=OH, 7=OH, 3'=OH, 5'=OH)
Fisetin (7=OH,3'=OH
Isohamnetin (3'=OCH3)
   sowie deren 3- und 7-Glycoside.

Alle diese Verbindungen besitzen aufgrund ihere natürlichen Herkunft eine hohe Akzeptanz als Inhaltssoffe kosmetischer und pharmazeutischer Mittel und kommen in der Natur in zahlreichen Beerenobstarten, Citrusfrüchten sowie anderen Obst- und Gemüsesorten und in einigen Kräuterpflanzen vor.

Die farbstabilisierende Wirkung dieser Substanzen ist vor allem deshalb überraschend, da andere Vertreter der Stoffgruppe der pflanzlichen Polyphenole entweder nicht farbstabilisierend auf sorbatkonservierte kosmetische oder pharmazeutische Mittel wirken, oder unter ungünstigen Bedingungen, wie z.B. intensiver Tageslichtbestrahlung, sogar zu einer Beschleunigung unerwünschter Verfärbungsreaktionen führen. Beispiele für Verbindungen, die auf sorbathaltige kosmetische Mittel nicht farbstabilisierend wirken sind die großen Gruppen der Hydroxyzimtsäuren, Hydroxycumarsäuren und Hydroxybenzoesäuren sowie deren natürlich vorkommende Ester mit Chinasäure, Shikimisäure, Äpfelsäure, Weinsäure und myo-Inosit. Einige dieser Verbindungen wirken sogar verstärkend auf unerwünschte Verfärbungen.

Als Beispiele seien hier Ferualsäure sowie Rosmarinsäure genannt, welche häufig aufgrund einer ihnen zugeschriebenen antioxidativen Wirkung in kosmetischen Mitteln verwendet werden, bezüglich der Farbstabilisierung sorbathaltiger Produkte jedoch keine positive Wirkung zeigen.

Auch synthetische Antioxidantien wie Propylgallat oder BHA wirken auf sorbathaltige kosmetische oder pharmazeutische Formulierungen nicht farbstabilisierend.

Um so bemerkenswerter ist das dazu unterschiedliche Verhalten flavonoider Verbindungen sowie insbesondere deren 3-Glycoside und 7-Glycoside.

Während nicht zum Ausdruck gebracht werden soll, daß die Erfindung auf den nachfolgend beschriebenen Wirkmechanismus beschränkt ist, so kann dennoch folgende Erklärung vermutet werden: Die farbstabilisierende Wirkung könnte zum einen in den günstigeren Löslichkeitseigenschaften (bessere Wasserlöslichkeit durch den größeren Anteil hydrophiler Strukturen im Molekül), aber ebenso in einer sterischen Hinderung von Oxidations- bzw. Polymerisationsaktionen an den phenolischen Gruppen des Moleküls begründet sein. Beides sind Eigenschaften, die antioxidativ wirkende Verbindungen nicht notwendigerweise besitzen und deren Fehlen mithin ihrer Wirksamkeit in anderen Systemen keineswegs abträglich ist, denen jedoch überraschenderweise für die vorliegende Problemstellung eine besondere Bedeutung zuzukommen scheint.

Das Flavonoid und/oder sein 3- bzw. 7-Glycosid werden dem Mittel - insbesondere dem kosmetischen bzw. pharmazeutischen Mittel - vorteilhaft in einer Konzentration von 0,005 bis 10 Gewichtsprozent zugesetzt, insbesondere 0,01 bis 2 Gewichtsprozent, bevorzugt 0,01 bis 1 Gewichtsprozent, besonders bevorzugt 0,015 bis 0,2 Gewichtsprozent bezogen auf die Gesamtmasse des Produkts. Bei den bevorzugten Einsatzkonzentrationen sind Flavonoide selbst nur sehr schwach farbgebend.

Die sorbatkonservierten Mittel, insbesondere die kosmetischen und pharmazeutischen Mittel enthalten zwischen 0,005 und 5 Gewichtsprozent Sorbinsäure oder eines derer Salze, insbesondere zwischen 0,05 und 2 Gewichtsprozent, bevorzugt zwischen 0,1 und 0,8 Gewichtsprozent, besonders bevorzugt zwischen 0,15 und 0,4 Gewichtsprozent.

Im folgenden wird die Erfindung anhand von Beispielen näher erläutert:

Eine sorbatkonservierte kosmetische Modellformulierung (Haarshampoo) ohne Verdickungszusatz wurde nach folgender Grundrezeptur hergestellt:

| | |
|---|---|
| Sodium lauryl myristyl ether sulfate (28%) | 22,4 g |
| Disodlum laureth sulfosuccinate (40%) | 9,6 g |
| Cocamido propyl betaine (30%) | 10,0 g |
| Polysorbate 20 (100%) | 1,6 g |
| Potassium sorbate | 0,5 g |
| Citric acid | 1,9 g |
| Triethanolamine | ad pH 5,0 |
| Aqua | ad 100 ml |

Das so hergestellte Produkt wurde mit verschiedenen Zusätzen vermischt, um deren farbstabitisierende Wirkung zu untersuchen. Zur Quantifizierung der Schutzwirkung dieser Zusätze gegenüber unerwünschten Produktverfärbungen kam folgende Versuchsanordnung zum Einsatz.

Das zu untersuchende Produkt wurde in ein oben offenes Behältnis gefüllt und für 20-30 Tage unter einer starken Lichtquelle (366nm) gelagert. Die Temperatur des Produkts während der Lagerung betrug dabei ca. 30 °C. Während dieser Lagerzeit wurden die jeweiligen Produkte mehrfach mittels eines Farbmeßgerätes (Minolta Chromameter) auf die eingetretene Verfärbung vermessen. Aus den Meßwerten, ausgedrückt in ^{Δ}E, wurde mittels einer Regressionsanalyse die mittlere tägliche Zunahme des Farbwertes berechnet und als Differenz zu den entsprechenden täglichen Zunahmen des Farbwertes einer Kontrollprobe (ohne Zusätze) angegeben. Damit drücken positive Werte eine beschleunigte, negative Werte eine verminderte Verfärbung des Modeliproduktes an.

Im einzelnen ergaben sich damit für die getesteten Zusätze folgende Ergebnisse:

| Erfindungsgemäß: | |
|---|---|
| Rutin (0,025%) | -0,19 |

| Vergleichsbeispiel: | |
|---|---|
| Allantoine (0,5%) | -0,09 |
| Urea(5%) | 0,03 |
| Propylgallate (0,05%) | 0,22 |
| BHA (0,02%) | 0,36 |
| Tocopheryl acetate (0,05%) | -0,02 |
| Ferulic acid (0,1%) | 0,02 |
| Rosmarinic acid (0,06%) | 0,11 |
| Phytic acid (0,1%) | 0,29 |
| EDTA (0,1%) | -0,12 |
| Iminodisuccinate-Na-salt (0,1%) | 0,00 |
| Sodium hexametaphosphate (0,3 %) | 0,06 |

Aus diesen Ergebnissen ist ersichtlich, daß Rutin, als Vertreter der Substanzklasse der glycosylierten Flavonoide bereits in einer Dosierung von 0,025 Gewichtsprozent eine deutliche Schutzwirkung gegenüber der sorbatabhängigen, licht- und wärmeinduzierten Verfärbung kosmetischer Mittel aufweist. Diese Wirkung übertrifft die farbstabilisierende Wirkung von Allantoin sowie die von EDTA deutlich. Andere phenolische Pflanzeninhaltssoffe wie Ferulasäure oder Rosmarinsäure zeigen praktisch keine oder sogar eine verfärbungsfördernde Wirkung. Häufig gebrauchte synthetische Antioxidantien wie BHA und Propylgallat verstärken ebenfalls die Verfärbungsneigung des sorbatkonservierten Systems, statt sie, wie aufgrund ihres antioxidativen Potentials vermutet werden könnte, zu vermindern.

## Patentansprüche

1. Mittel, enthaltend Sorbinsäure und/oder ein oder mehrere Sorbate sowie ein Flovonoid und/oder Flavonoidderivat.

2. Mittel nach Anspruch 1, dadurch gekennzeichnet, daß das Sorbat ein Natrium-, Kalium- und Calciumsorbat ist.

3. Mittel nach Anspruch 1 oder 2, dadurch gekennzeichnet, daß das Flavonoid oder Flavonoidderivat eine Verbindung der Formel (I) ist 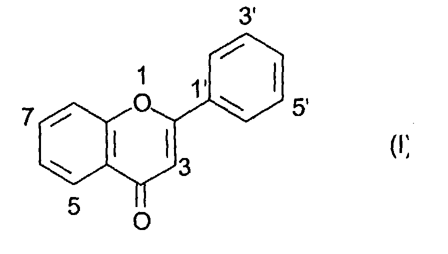 mit 3=H(Flavone) oder 3=OH, 4'=OH(Flavonole).

4. Mittel nach Anspruch 3, dadurch gekennzeichnet, daß das Flavonoid oder Flavonoidderivat eine Verbindung der Formel (I) ist mit folgenden Substituenten:
Flavone:
Apigenin (5=OH, 7=OH)
Luteolin (5=OH, 7=OH, 3'=OH)
Diosmetin (4'=OCH₃, 3'=OH)
Chrysoeriol (3'=OCH₃, 4'=OH)
Flavonole:
Kämpferol (5=OH, 7=OH)
Quercetin (5=OH, 7=OH, 3'=OH)
Morin (5=OH, 7=OH, 2'=OH)
Robinetin (7=OH, 3'=OH, 5'=OH)
Gossypetin (5=OH, 7=OH, 3'=OH, 8=OH)
Myricetin (5=OH, 7=OH, 3'=OH, 5'=OH)
Fisetin (7=OH,3'=OH)
Isohamnetin (3'=OCH3)
sowie deren 3- und 7-Glycoside.

5. Mittel nach einem der Ansrüche 1 bis 4, dadurch gekennzeichnet, daß das Flavonoid und/oder Flavonoidderivat in dem Mittel in einer Menge von 0,005 bis 10 Gew.-% (bezogen auf das Gewicht des Mittels) enthalten ist.

6. Mittel nach einem der Ansrüche 1 bis 5, dadurch gekennzeichnet, daß die Sorbinsäure und/oder das (die) Sorbat(e) in dem Mittel in einer Menge von 0,005 bis 5 Gew.-% (bezogen auf das Gewicht des Mittels) enthalten ist.

7. Verfahren zur Lichtstabilisierung von sorbatkonservierten Mitteln, dadurch gekennzeichnet, daß man dem Mittel ein Flavonoid und/oder ein Flavonoidderivat zusetzt.

8. Verwendung eines Flavonoids und/oder Flavonoidderivats zur Lichtstabilisierung von sorbathaltigen Mitteln.

9. Verwendung eines Mittels nach einem der Ansprüche 1 bis 6 als pharmazeutisches Mittel.

10. Verwendung eines Mittels nach einem der Ansprüche 1 bis 6 als kosmetisches Mittel.
